(19) Europäisches Patentamt
European Patent Office
Office européen des brevets

(11) EP 1 650 210 A1

(12) # EUROPEAN PATENT APPLICATION

published in accordance with Art. 158(3) EPC

(43) Date of publication:
**26.04.2006 Bulletin 2006/17**

(21) Application number: **04747591.8**

(22) Date of filing: **15.07.2004**

(51) Int Cl.:
***C07D 493/10*** *(1985.01)* ***A61K 31/35*** *(1985.01)*
***A61P 19/08*** *(2000.01)* ***A61P 19/10*** *(2000.01)*

(86) International application number:
**PCT/JP2004/010125**

(87) International publication number:
**WO 2005/005433 (20.01.2005 Gazette 2005/03)**

(84) Designated Contracting States:
**AT BE BG CH CY CZ DE DK EE ES FI FR GB GR HU IE IT LI LU MC NL PL PT RO SE SI SK TR**

(30) Priority: **15.07.2003 JP 2003197229**

(71) Applicants:
- **RIKEN**
  **Wako-shi,**
  **Saitama 351-0198 (JP)**
- **Chubu University Educational Foundation**
  **Nagoya-shi,**
  **Aichi 460-0012 (JP)**

(72) Inventors:
- **OSADA, Hiroyuki**
  **3510198 (JP)**
- **MACHIDA, Kiyotaka**
  **3510198 (JP)**
- **SHIMIZU, Takeshi**
  **3510198 (JP)**
- **NAKATA, Tadashi**
  **3510198 (JP)**
- **SHINKI, Toshimasa**
  **1420064 (JP)**
- **WOO, Je-Tae,**
  **Chubu University Educational Found.**
  **Kasugai-shi,**
  **Aichi,**
  **487-8501 (JP)**
- **NAGAI, Kazuo,**
  **Chubu University Educational Found.**
  **Kasugai-shi,**
  **Aichi,**
  **487-8501 (JP)**

(74) Representative: **Bannerman, David Gardner et al**
**Withers & Rogers LLP**
**Goldings House,**
**2 Hays Lane**
**London SE1 2HW (GB)**

(54) **THERAPEUTIC AGENT FOR HYPERPOTASSEMIA AND BONE DISEASE**

(57) The present invention has an object to provide a novel therapeutic agent for hypercalcemia and bone diseases, and a novel compound capable of being used as an active ingredient of the therapeutic agent.

The present invention is a therapeutic agent for hypercalcemia or bone diseases, comprising as an active ingredient a reveromycin A derivative represented by the general formula (I) or a pharmaceutically acceptable salt thereof.

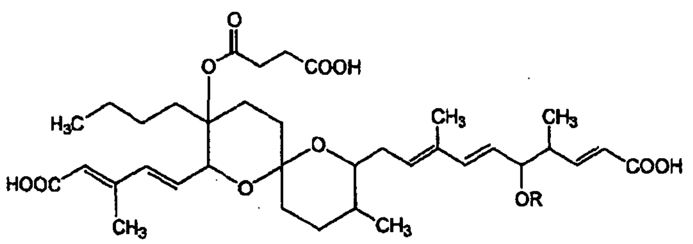

(I)

(R represents a hydroxyl-protecting group releasable under acidic conditions.)

EP 1 650 210 A1

## Description

TECHNICAL FIELD

The present invention relates to a novel therapeutic agent for hypercalcemia and bone diseases.

BACKGROUND ART

The mass and function of a bone are maintained by a balance between osteogenesis by osteoblasts and bone resorption (bone destruction) by osteoclasts. The osteoporosis is a disorder associated with a reduced bone mass due to disruption of a balance in bone metabolism caused by an excess bone resorption by activated mature osteoclasts, and the number of patients suffering from osteoporosis is increasing rapidly in aging society.

As a bone resorption inhibitor to be used as a therapeutic agent of osteoporosis, for example, there is estrogen, one of female hormones at present. In addition, as a therapeutic method, there is a method of direct administration of such an agent. However, such a method has a disadvantage in that it may cause serious side effects. Furthermore, estrogen may inhibit the bone resorption by facilitating the secretion of calcitonin, one of thyroid hormones, so that pharmaceutical preparations of peptide hormones such as calcitonin or the like have been used as bone resorption inhibitors. However, such peptide hormone preparations have disadvantages in that they have short durability in inhibiting effects on bone resorption and difficulties in use for the patients having hypersensitive conditions such as allergy. Therefore, the development of a novel therapeutic agent for bone diseases, which takes the place of any of them, has been desired.

Non-hormonal drugs which can directly inhibit the functions of osteoclasts causing the bone resorption, are expected to be clinical drugs with little side effects. Therapeutic agents for bone diseases comprising reveromycins as active ingredients have been known as the non-hormonal therapeutic agents for bone diseases (see JP 07-223945 A). Nevertheless, the development of another therapeutic agent for bone diseases has been desired.

As described above, within a living body, the cause of bone diseases associated with a decrease in bone density is excessive bone resorption by osteoclasts. For instance, within a living body, a parathyroid hormone (PTH) induces the activation of mature osteoclasts as well as facilitates the differentiation of osteoclasts. Therefore, the development of a drug that acts on osteoclasts on which the PTH effects (e.g., a drug that inhibits the formation of osteoclasts responsible for the function of bone resorption or a drug that inhibits the function of osteoclasts) have been largely expected in clinical applications as a therapeutic agent for bone diseases such as osteoporosis and a therapeutic agent for hypercalcemia or the like.

DISCLOSURE OF THE INVENTION

The present invention meets the desires described above, and has an obj ect to provide a novel therapeutic agent for hypercalcemia and bone diseases, and a novel compound capable of being used as an active ingredient of the therapeutic agent.

As a result of extensive studies for solving the above objects, the inventors of the present invention have newly found that a specific derivative of a natural compound reveromycin A and reveromycin A derivatives newly developed on the basis of reveromycin A selectively induce the cell death of the mature osteoclasts to inhibit the action of the bone resorption. In addition, they have also found a new fact that those reveromycin A derivatives can effectively inhibit PTH-depending bone resorption, which is the most important bone resorption system in a living body. From such findings, the inventors of the present invention have found that those reveromycin A derivatives can be used as therapeutic agents for hypercalcemia and bone diseases, and completed the present invention.

That is, the gist of the present invention is as follows.

(1) A therapeutic agent for hypercalcemia or bone diseases, comprising as an active ingredient a reveromycin A derivative represented by the general formula (I) or a pharmaceutically acceptable salt thereof.

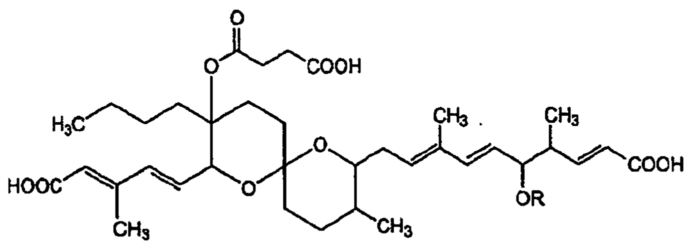

(I)

(R represents a hydroxyl-protecting group releasable under acidic conditions.)

(2) The therapeutic agent according to (1), wherein R is a tert-butyldimethylsilyl group in the general formula (I).

(3) A reveromycin A derivative represented by the general formula (II) or a pharmaceutically acceptable salt thereof.

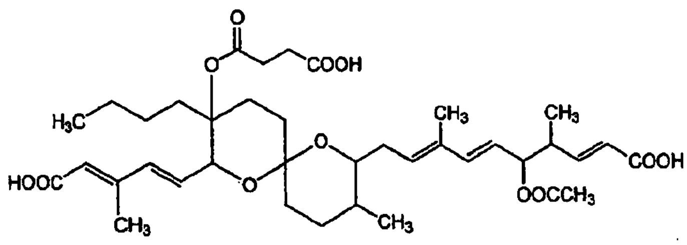

(II)

(4) A therapeutic agent for hypercalcemia or bone diseases, comprising as an active ingredient the reveromycin A derivative or the pharmaceutically acceptable salt thereof according to (3).

BEST MODE FOR CARRYING OUT THE INVENTION

Hereinafter, the present invention will be described in detail.

(1) Reveromycin A derivative to be used in the therapeutic agent of the present invention

A reveromycin A derivative to be used in the therapeutic agent of the present invention is a compound represented by the following general formula (I) and a pharmaceutically acceptable salt thereof.

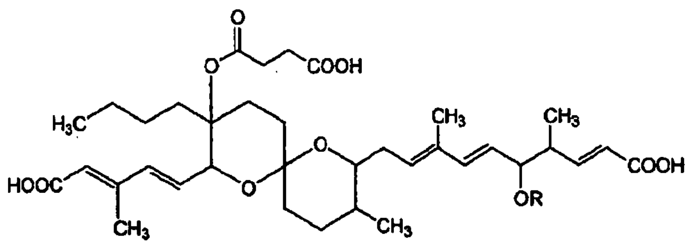

(I)

In the formula, R represents a hydroxyl-protecting group releasable under acidic conditions.

In the present invention, the term "hydroxyl-protecting group" is not particularly limited as far as it is an atomic group capable of temporarily protecting a hydroxyl group from any of various reactions.

Furthermore, the phrase "releasable under acidic conditions" means that the protective group can be released under acidic conditions but more difficult to be released under neutral conditions compared with the acidic conditions. Preferably, the phrase "releasable under acidic conditions" means that the protective group can be released under acidic environments generated by the mature osteoclasts or the like but more difficult to be released under neutral environments in which normal cells exist. Furthermore, the phrase "more difficult to be released under neutral conditions" means that the protective group is stable and hardly released under neutral conditions. Specifically, the phrase "releasable under acidic conditions" means that the protective group will be released under acidic conditions of pH 4.0 or less, but hardly released under neutral conditions of pH7.0. The protective group "releasable under acidic conditions" to be used in the present invention can be chosen, for example, by the following procedure. A reveromycin A derivative having a hydroxy

group at position 5 protected by the protective group to be examined is administered to cells that generate any acidic condition such as mature osteoclasts, and cells that exist under a neutral condition such as osteoclast precursor cells. Then, the cell-death inducing activity is measured by the measurement of the number of living cells or the MTT method or the like to select a derivative having a cell-death inducing activity in the cells generating an acidic environment higher than in the cells existing under a neutral condition.

An acyl group, alkyl group, silyl group, or the like can be used for R, and an acyl group or silyl group can be preferably used. More specific examples of an acyl group that can be used include an alkyl carbonyl group such as a formyl group, acetyl group, propionyl group, butyryl group, isobutyryl group, valeryl group, isovaleryl group, and hexanoyl group. More specific examples of an alkyl group that can be used include: an alkyl group such as a methyl group and ethyl group; a tetrahydropyranyl group; an alkoxyalkyl group such as an ethoxyethyl group and methoxymethyl group; an arylalkyl(aralkyl) group such as a benzyl group; and an alkylthioalkyl group such as a methylthiomethyl group. More specific examples of a silyl group that can be used include a tert-butyl diphenylsilyl group, tert-butyl dimethylsilyl group, triethylsilyl group, triisopropylsilyl group, and dimethylethylsilyl group. R is not limited to those examples.

A preferable embodiment of the reveromycin A derivative to be used in the therapeutic agent of the present invention is a reveromycin A derivative having the general formula (I) where R is a tert-butyldimethylsilyl group.

Another preferable embodiment of the reveromycin A derivative to be used in the therapeutic agent of the present invention is a novel reveromycin A derivative having the general formula (I) where R is an acetyl group.

In the therapeutic agent of the present invention, any of pharmaceutically acceptable salts of the reveromycin A derivative may be used. The pharmaceutically acceptable salts include, but not limited to, for example: mineral salts such as hydrochloride and sulfate; organic salts such as p-toluene sulfonate; metal salts of a sodium salt, potassium salt, calcium salt, and the like; an ammonium salt; organic ammonium salts such as a methyl ammonium salt; and amino acid salts such as a glycine salt.

The reveromycinA derivative represented by the general formula (I) has plural asymmetric carbons. In addition, there is a case having one additional asymmetric carbon or more depending on the kind of a substituent. A stereoisomer such as an optical isomer or a diastereomer based on any of those asymmetric carbons is present. In the present invention, a mixture of given stereoisomers, a racemic body, or the like as well as a stereoisomer having a pure configuration can be used. Furthermore, there are also reveromycin A derivatives having olefinic double bonds. Even though there are geometric isomers based on the double bonds, a mixture of given geometric isomers can be used in the present invention as well as geometric isomers having pure configurations. The reveromycin A derivative to be used in the present invention can exist as any crystal form, or may exist as a hydrated or solvated product. Those substances can be also used in the present invention.

Reveromycin A can be produced by any of methods known in the art or alternative methods based thereon, for example, those disclosed in JP 06-33271 B and Journal of Antibiotics vol. 45, No. 9, pp 1409-1413 (1992) in which reveromycin A is collected after culturing reveromycin-A-producing bacteria.

For the reveromycin A derivatives, the configuration of a specific reveromycin A derivative having an effective activity as a therapeutic agent of the present invention has been revealed by the present invention. Therefore, the reveromycin A derivatives can be produced by any of methods known in the art, such as a manufacturing method for a reveromycin A derivative disclosed in Bioorganic & Medicinal Chemistry Letters Vol. 12, pp 3363-3366 (2002) or a common organic syntheticprocedure . In addition, in the Examples of the present invention, specific examples of synthetic procedures of the compounds to be used as the therapeutic agent of the present invention are described and those synthetic procedures can be referred.

(2) Therapeutic agent for hypercalcemia and bone diseases of the present invention

The therapeutic agent of the present invention is a drug that contains a specific reveromycin A derivative or a pharmaceutically acceptable salt thereof as an active ingredient and can be used for the treatment and/or prevention of hypercalcemia and any of bone diseases. The bone diseases include both endogenic bone diseases such as a decrease in bone mass and exogenic bone diseases such as physical bone fractures. The agent of the present invention can be used for the treatment and/or prevention of the bone diseases mentioned above or used for shortening the duration of therapy of the bone diseases mentioned above. The endogenic bone diseases include all of disorders associated with excessive formation of osteoclasts and/or excessive function in a living body. Specific examples of the bone diseases include, but not limited to, osteoporosis, bone-disease-related hypercalcemia, bone Paget’s disease, osteoclastoma, osteosarcoma, arthropathy, chronic articular rheumatism, osteotis deformans, primary hyperthyroidism, osteopenia, osteoporosis, osteomalacia, traumatic bone fracture, fatigue bone fracture, and fragility of bone tissues, fractures, and the like due to other disorders such as nutrition disorder and malignant tumor. The therapeutic agent of the present invention can be preferably used for the treatment and/or prevention of hypercalcemia and bone diseases which are induced by vitamin $D_3$, IL-1, or the PTH.

The administration method, dosage form, and dosage of the therapeutic agent of the present invention can be

appropriately determined depending on general pharmaceutical techniques according to the purpose of use. For instance, for administration to an animal such as human for the purpose of the treatment or prevention, it can be orally administered as, for example, a powder, granule, tablet, capsule, pill, or solution agent, or parenterally administered as, for example, injection, suppository, percutaneous absorbent, or inhalant agent. The effective amount of the reveromycin A derivative to be used in the therapeutic agent of the present invention can be prepared as a pharmaceutical agent, if required, by mixing with any of pharmaceutical additives suitable for dosage form such as a filler, binder, humectant, disintegrator, lubricant, and the like depending on general pharmaceutical techniques. In the case of the injection, a pharmaceutical preparation is prepared by being subjected to a sterilization treatment together with an appropriate carrier. The dosage may vary depending on the disease condition, administration route, or age or weight of a patient, therefore it is conclusively left on a doctor's diagnosis. However, for orally administrating the amount of an effective ingredient to an adult, typically 20 to 500 mg/kg/day, preferably 50 to 300 mg/kg/day may be administered. For parenteral administration, 10 to 300 mg/kg/day, preferably 20 to 200 mg/kg/day may be administered. Alternatively, the dosage may be administered at once or in several divided doses.

In the present invention, various reveromycin A derivatives are chemically synthesized. Comparing with a natural-type reveromycin A, the development of a compound that inhibits the growth of mature osteoclasts more specifically in a cell culture system has been attempted. As a result, it is newly found that specific reveromycin A derivatives represented by the general formula (I), particularly one represented by the general formula (I) in which R is a tert-butyldimethylsilyl group and one in which R is an acetyl group show a selective effect to inhibit the growth of mature osteoclasts, compared with the natural reveromycin A. Inotherwords, a derivative in which the hydroxyl group at position 5 of reveromycin A is protected with an appropriate protective group has a slight cell-killing activity at a neutral pH level. In contrast, for the mature osteoclasts that produce an acidic condition, the derivative acts as a prodrug having a strong activity of apoptosis induction. Those derivatives represent excellent selective effects of apoptosis induction that the apoptosis inducing activities of those derivatives to cells other than mature osteoclasts is lowered. The selectivity to the activated mature osteoclasts is high, so that these reveromycin A derivatives may have little side effects.

In addition, as a result of the administration of specific reveromycin A derivatives to rats having hypercalcemia caused by administration of the PTH after extraction of their thyroid and parathyroid glands, it was newly found that those reveromycin A derivatives could effectively inhibit the bone resorption action to be induced by the PTH. In addition, the inhibitory effects of those reveromycin A derivatives on bone resorption action had a prolonged duration, compared with one with the conventional calcitonin.

In other words, the present invention has found reveromycin A derivatives capable of inducing selective cell death of the mature osteoclasts responsible for bone resorption. These reveromycin A derivatives could effectively inhibit the bone resorption due to PTH and the durability of such an effect could be increased, compared with that of calcitonin or the like. Therefore, these reveromycin A derivatives can be expected as effective therapeutic agents for bone diseases, hypercalcemia, and the like which are induced by excess activation of the osteoclasts or the like.

## EXAMPLES

Hereinafter, the present invention will be further described in detail with reference to Production Examples and Examples. However, the present invention is not limited to these examples.

Various reveromycin A derivatives were synthesized by the methods described in Bioorganic & Medicinal Chemistry Letters Vol. 12, pp 3363-3366 (2002) and the Production Examples described below. In addition, reveromycin A was produced by the method described in JP 06-33271 B. Twenty-nine different reveromycin A derivatives synthesized were listed in Tables 1 and 2 below, respectively. In Tables 1 and 2, "Ac" represents an acetyl group, "allyl" represents an allyl group, "Et" represents an ethyl group, "TBS" represents a tert-butyldimethylsilyl group, "Me" represents a methyl group, and "MTM" represents a methylthiomethyl group.

Table 1

| Comp. | Structure | Comp. | Structure |
|---|---|---|---|
| Revero-mycin A | 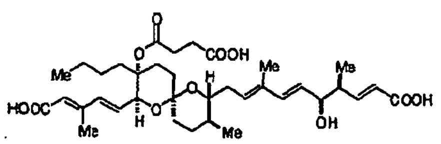 | 6 | 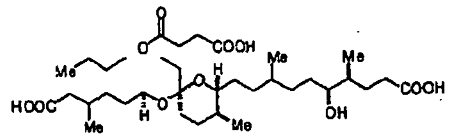 |
| 1 | 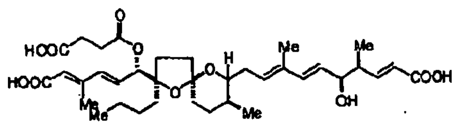 | 7 | 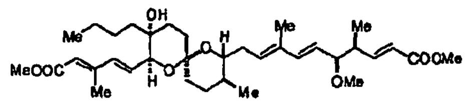 mixture with 5-CHO |
| 2 | 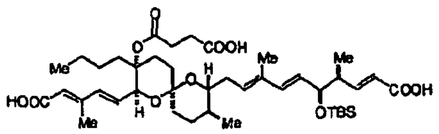 | 8 | 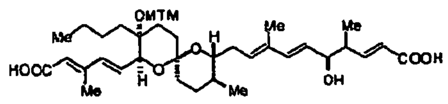 |
| 3 | 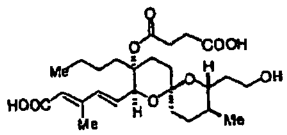 | 9 | 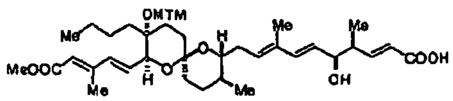 |
| 4 | 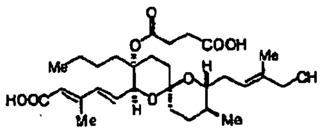 | 10 | 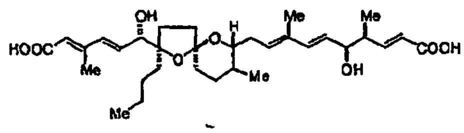 |
| 5 | 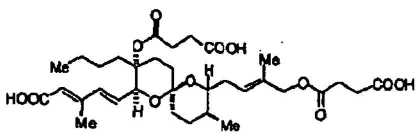 | 11 | 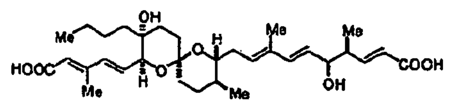 mixture with 5,6-spiroacetal |
| 1390-II | 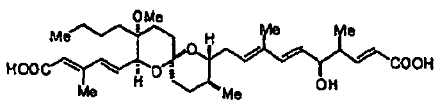 | 12 | 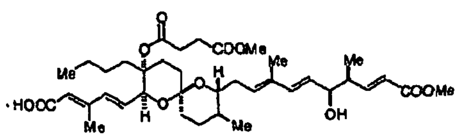 |
| 1392-II | 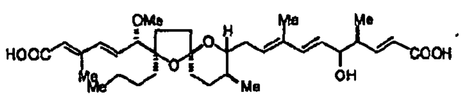 | 13 | 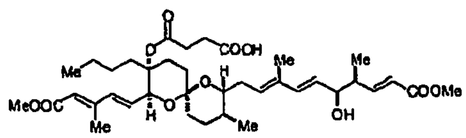 |

Table 2

| Comp. | Structure | Comp. | Structure |
|---|---|---|---|
| 14 | 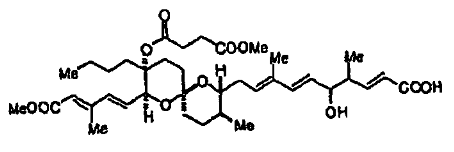 | 21 | 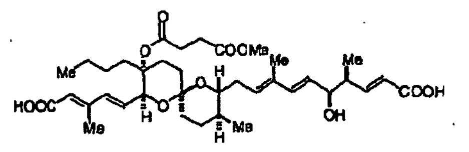 |
| 15 | 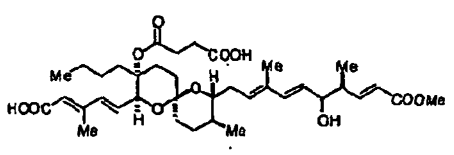 | 22 | 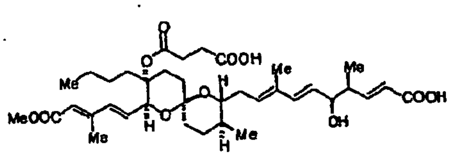 |
| 16 | 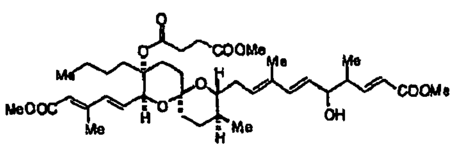 | 23 | 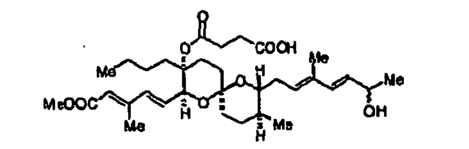 |
| 17 | 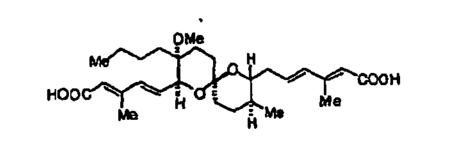 | 24 | 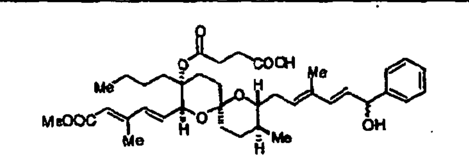 |
| 18 | 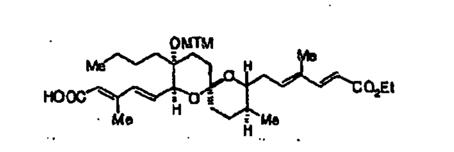 | 25 | 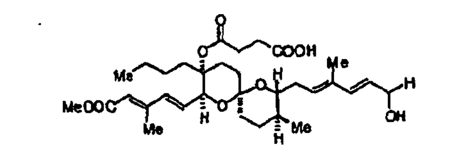 |
| 19 | 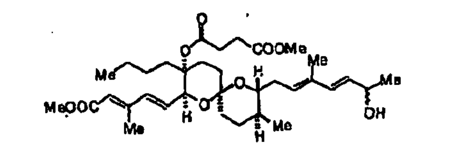 | 26 | 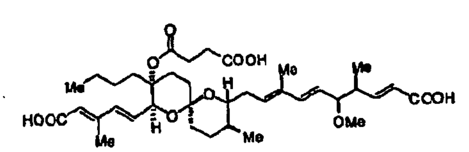 |
| 20 | 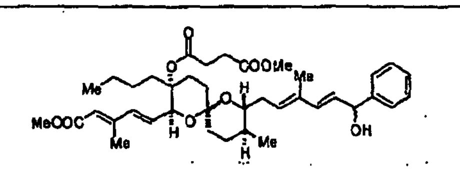 | 27 | 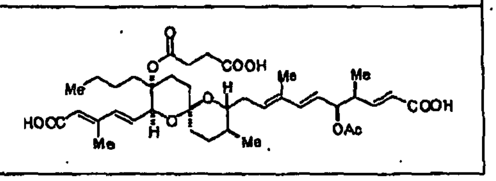 |

Hereinafter, the production examples of Compounds 2 and 27 will be described, respectively.

<Production Example 1> Synthesis of Compound 2 (C5-silylether) Synthesis of C5-silylether (Compound 2)

Under a nitrogen atmosphere, imidazole (13.6 mg, 0.2 mmol) and TBSCl (t-butyldimethyl silylchrolide) (18.0 mg, 0.12 mmol) were added to reveromycin A (6.6 mg, 0.01 mmol) in DMF (dimethyl formamide (300 $\mu$l) solution at room temperature and then stirred overnight, followed by dilution with ethyl acetate (AcOEt). The resulting organic layer was washed at 0° C sequentially with 1N HCl and saturated brine. After removing the solvent, methanol (MeOH): tetrahydrofuran (THF):$H_2O$ = 3:2:1 (600 $\mu$l) and $K_2CO_3$ (5 mg) were added to the residue and then stirred for 1 hour at room temperature. After removing the solvent, the residue was purified by $SiO_2$ column chromatography (n-hexane: AcOEt = 1:1, 1% acetic acid), thereby obtaining 5-silylether (Compound 2) (colorless oily product: 5.0 mg, 71%).

$^1H$ NMR (500 MHz, $CD_3OD$) $\delta$ = 0.06 (s, 3H), 0.10 (s, 3H), 0.85 (d, J = 6.4 Hz, 3H), 0.89 (t, J = 6.9 Hz, 3H), 0.95 (s, 9H), 1.09 (d, J = 6.4 Hz, 3H), 1. 79 (s, 3H), 2.31 (s, 3H), 2.63 (m, 4H), 3.47 (ddd, J = 10.0, 5.0, 5.0 Hz, 1H), 4.26 (dd, J = 6.9, 5.5 Hz, 1H), 4.65 (brd, J = 8.2 Hz, 1H), 5.54 (dd, J = 15.6, 6.9 Hz, 1H), 5.63 (dd, J = 7.3, 7.3 Hz, 1H), 5.82

(d, J = 15.6 Hz, 1H), 5.92 (brs, 1H), 6.27 (d, J = 15.6 Hz, 1H), 6.46 (m, 2H), 7.03 (dd, J = 15.6, 7.3 Hz, 1H).

$^{13}$C NMR (125 MHz, $CD_3OD$) δ = 14.2, 14.4, 14.9, 18.1, 19.1, 23.9, 25.1, 25.5, 26.4, 28.6, 29.9, 31.1, 32.8, 33.3, 35.3, 36.9, 44.9, 76.2, 78.1, 79.9, 84.3, 97.0, 121.5, 122.3, 128.6, 129.4, 134.1, 135.6, 137.3, 139.3, 152.6, 153.5, 170.2, 170.2, 173.4, 175.9.

<Production Example 2> Synthesis of Compound 27 (C5-acetate)

(i) Synthesis of triallyl ester

Reveromycin A (66.1 mg, 0.1 mmol), allyl alcohol (700 μl), EDCI (1-ethyl-3(3-dimethylaminopropyl) carbodiimide (178.2 mg, 0.6 mmol), and DMAP (4-dimethyminopyridinela) (1.2 mg, 0.01 mmol) were placed in a 2 ml container made of Teflon (registered trademark of DuPont Corp.). Subsequently, the container was filled with anhydrous $CH_2Cl_2$ and then pressurized at 1.5 GPa for 2 days at room temperature in a high pressure equipment. The reaction mixture was diluted with diethylether ($Et_2O$) and then washed sequentially with a 2N $Na_2CO_3$ aqueous solution and saturated brine, followed by drying over $MgSO_4$ and removing the solvent. The residue was purified by column chromatography (n-hexane:$Et_2O$ = 2:1), thereby obtaining triallyl ester (28.1 mg, 36%).

$^1$H NMR (500 MHz, $CDCl_3$) δ = 0.76 (d, J = 6.4 Hz, 3H), 0.82 (t, J = 6.9 Hz, 3H), 1.09 (d, J = 6.9 Hz, 3H), 1.72 (s, 3H), 2.25 (brs, 3H), 2.66 ( m, 4H), 3.41 (ddd, J = 9.9, 4.8, 4.8 Hz, 1H), 4.11 (dd, J = 7.3, 5.6 Hz, 1H), 4.61 (d, J = 8.0 Hz, 1H), 4.62 (m, 6H), 5.24 (dd, J = 10.4, 1.4 Hz, 2H), 5.24 (dd, J = 10.4, 1.4 Hz, 1H), 5.32 (ddd, J = 17.2, 1.4, 1.4 Hz, 1H), 5.34 (ddd, J = 17.2, 1.4, 1.4 Hz, 1H), 5.35 (ddd, J = 17.2, 1.4, 1.4 Hz, 1H), 5.51 (dd, J = 15.6, 7.3 Hz, 1H), 5.54 (brt, J = 7.3 Hz, 1H), 5.86 (brs, 1H), 5.90 (d, J = 16.0 Hz, 1H), 5.96 (m, 3H), 6.22 (d, J = 15.6 Hz, 1H), 6.34 (d, J = 15.6 Hz, 1H), 6.38 (dd, J = 15.6, 8.0 Hz, 1H), 7.04 (dd, J = 16.0, 7.5 Hz, 1H).

$^{13}$C NMR (125 MHz, $CDCl_3$) δ = 12.6, 14.1, 14.4, 14.7, 17.7, 22.9, 29.4, 30.2, 33.9, 34.2, 36.0, 42.7, 64.8, 65.1, 65.5, 74.9, 76.1, 78.2, 83.1, 95.8, 118.1, 118.1, 118.3, 119.7, 121.4, 126.0, 129.4, 132.0, 132.3, 132.3, 133.2, 133.8, 137.4, 137.7, 150.9, 151.9, 166.1, 166.4, 171.2, 171.8.

(ii) Synthesis of 5-acetate

Under a nitrogen atmosphere, pyridine (4.4 μl, 54.4 μmol), DMAP (1 piece) , and acetic anhydride ($Ac_2O$) (2.1 μl, 21.8 μmol) were added sequentially to triallyl ester (8.5 mg, 10.9 μmol) in $CH_2Cl_2$ solution (1 ml) and then stirred at 0°C for 3 hours. After removing the solvent, the residue was purified by column chromatography (n-hexane:AcOEt = 3 : 1), thereby obtaining 5-acetoxytriallyl ester (colorless oily product: 6.8 mg, 76%).

$^1$H NMR (500 MHz, $CDCl_3$) δ = 0.74 (d, J = 6.4 Hz, 3H), 0.83 (t, J = 6.9 Hz, 3H), 1.08 (d, J = 6.9 Hz, 3H), 1.70 (bs, 3H), 2.07 (s, 3H), 2.28 (d, J =0.9 Hz, 3H), 2.67 (m, 4H), 2.77 (m, 4H), 3.43 (dt, J = 10.1, 3.7 Hz, 1H), 4.60 (ddd, J = 6.0, 1.4, 1.4 Hz, 2H), 4.62 (d, J= 8.7 Hz, 1H), 4.64 (ddd, J = 6.0, 1.4, 1.4 Hz, 2H), 4.65 (ddd, J = 6.0, 1.4, 1.4 Hz, 2H), 5.24 (ddt, J = 10.5, 1.4, 1.4 Hz, 1H), 5.25 (ddt, J= 10.5, 1.4, 1.4 Hz, 1H), 5.25 (ddt, J = 10. 5, 1.4, 1.4 Hz, 1H), 5.27 (dd, J=8.3, 5. 5 Hz, 1H), 5.32 (ddt, J = 17.2, 1.4, 1.9 Hz, 1H), 5.34 (ddt, J = 17.2, 1.4, 1.4 Hz, 1H), 5.39 (ddt, J = 17.2, 1.4, 1.4 Hz, 1H), 5.38 (dd, J = 15.6, 8.3 Hz, 1H) , 5.64 (brt, J=6.9 Hz, 1H), 5.86 (brs, 1H) , 5. 87 (dd, J = 15. 6, 0.9 Hz, 1H), 6.30 (d, J = 15.6 Hz, 1H), 6.33 (d, J = 15.6 Hz, 1H), 6.38 (dd, J = 15. 6, 8.7 Hz, 1H) , 6.99 (dd, J = 15. 6, 7.3 Hz, 1H) , 7.03 (dd, J = 15.6, 7.3 Hz, 1H).

$^{13}$C NMR (125 MHz, $CDCl_3$) δ = 12.7, 14.0, 14.4, 15.1, 17.7, 21.4, 22.8, 29.3, 30.2, 35.8, 40.6, 64.8, 65.2, 65.5, 74.5, 77.6, 78.2, 83.2, 95.9, 118.2, 118.3, 118.5, 120.0, 121.0, 121.9, 130.0, 132.1, 132.3, 132.5, 133.3, 133.9, 137.8, 139.8, 149.6, 151.8, 166.2, 166.6, 170.2, 171.4, 172.0.

Under a nitrogen atmosphere, tetrakis (triphenylphosphine) palladium ($Pd(Ph_3P)_4$) (0.41mg, 0.35μmol), triphenylphosphine ($Ph_3P$) (0. 19 mg, 0.7 μmol) , and pyrrolidine (7.3 μl, 0.086 mmol) were added sequentially to 5-acetoxytriallyl ester (5.8 mg, 0.07 μmol) in $CH_2Cl_2$ (1 ml) solution at 0 °C and then stirred for 3 hours. The reaction solution was diluted with EtOAc and extracted with 2N $Na_2CO_3$. The water layer was neutralized with 2N HCl and then extracted with EtOAc. After washing the organic layer with saturated brine, the organic layer was dried over $MgSO_4$ and the solvent was then removed, thereby obtaining a 5-acetate (4.2 mg, 85%).

$^1$H NMR (500 MHz, $CDCl_3$) δ = 0.83 (d, J = 6.4 Hz, 3H), 0.89 (t, J = 6.9 Hz, 3H), 1.13 (d, J = 6.9 Hz, 3H), 1.78 (brs, 3H), 2.11 (s, 1H), 2.30 (d, J= 1.4 Hz, 3H), 2.63 (m, 4H), 3.99 (dt, J = 10.1, 5.5 Hz, 1H), 4.66 (d, J = 6.9 Hz, 1H), 5.32 (dd, J = 7.3, 6.0 Hz, 1H), 5.51 (dd, J = 15.6, 7.8 Hz, 1H), 5.69 (t, J = 7.3 Hz, 1H), 5.88 (d, J = 15.6 Hz, 1H), 5.92 (brs, 1H), 6.33 (d, J = 15.6 Hz, 1H), 6.46 (d, J = 15.6 Hz, 1H), 6.48 (dd, J = 15.6, 6.9 Hz, 1H), 6.95 (dd, J = 15.6, 7.3 Hz, 1H).

$^{13}$C NMR (125 MHz, $CDCl_3$) δ = 12.8, 14.2, 14.7, 15.2, 18.0, 23.8, 25.1, 25.5, 28.7, 29.9, 31.1, 32.8, 32.9, 34.7, 35.2, 36.9, 41.8, 76.2, 78.8, 79.8, 84.3, 97.1, 121.6, 122.9, 123.4, 130.9, 134.1, 135.3, 139.3, 140.3, 151.0, 152.5, 170.2, 170.2, 173.4, 176.0.

<Example 1> Investigation on cell-death inducing activities of reveromycin A and reveromycin A derivatives on mature osteoclasts

The inhibitory effects of reveromycin A and the derivatives thereof on the growth of the mature osteoclasts, which is a causing factor of bone resorption, were investigated, respectively.

Using 29 different reveromycin A derivatives synthesized as described above, the cell-death inducing activities on the mature osteoclasts and undifferentiated macrophage-like cells derived from amouse (RAW264 cells) were examined, respectively. The RAW264 cells, a mouse-macrophage cell line, were suspended in a 10% FBS-αMEM (manufactured by Gibco BRL Co., Ltd.) and then 12,000 cells/100 μl were inoculated in a 96-well plate. After 1-hour incubation, 100 μl of a culture medium containing RANKL (100 μg/ml) and PD98059 (40 μM) were added (final concentration: RANKL 50 μg/ml and PD98059 20 μM) and then incubated for 4 days to allow the differentiation of the cells to mature osteoclasts (J. Biol. Chem. Vol. 277, pp. 47366-47372 (2002)). Furthermore, on the second day of the culture, the half of the culture medium was replaced with new one. From each well of the plate where the cells were differentiated to the mature osteoclasts, 100 μl/well of the culture medium was removed. In addition, 100 μl of a culture medium that contains a two-fold concentration of reveromycin A derivative was added and then incubated for 24 hours, followed by staining with TRAP (tartarate resistant acid phosphatase) to calculate the number of multinuclear osteoclasts remained, and the cell death (apoptosis) inducing activity ($ED_{50}$ level) was then calculated. The apoptosis inducing activity was confirmed by the observation of agglutination of nuclei stained with 10 μM Hoechst 33258 for 10-minututes.

The cell-death inducing activities to the undifferentiated RAW264 cells were investigated by the MTT method (J. Immunol. Methods, Vol. 65, pp 55-63 (1983). The RAW 264 cells were plated on a 96-well plate at a density of 40,000 cells/100 μl and then incubated for 24 hours. After the 24-hour incubation, 50 μl of a culture medium containing a three-fold concentration of the drug was added and then incubated for 24 hours to investigate the cell-death inducing activity. Two hours before the measurement of the cell-death inducing activity, 15 μl of 0.5% MTT-PBS (phosphate buffer saline) was added and incubated. After that, the residue from which the culture solutionwas removed was dissolved in 100 μl of DMSO (dimethyl sulfoxide) and the absorbance at 570 nm (a reference wave length of 630 nm) was measured, followed by calculating the $ED_{50}$ level of the cell-death inducing activity.

As a result of the experiment, as shown in Table 3, the $ED_{50}$ level of the cell-death inducing activity of reveromycin A to the mature osteoclasts was 0.18 μM. Meanwhile, the $ED_{50}$ level of the cell-death inducing activity to the RAW264 cells was 24 μM, so that reveromycinAwill act on the mature osteoclasts with high selectivity. On the other hand, the reveromycin A derivatives (Compound 2 and Compound27) do not show any growth inhibitory action on the osteoclast precursor cells RAW264 even at a comparatively high concentration while it showed a selective apoptosis inducing effect on the mature osteoclasts. Each of the reveromycin A derivatives 2 (C5-sillyl ether) and 27 (C5-acetate) was a compound modified at the hydroxy group at position 5, but showed the selective induction of cell death to the mature osteoclasts. Proton secretion by the mature osteoclasts causes an acidic environment around the mature osteoclasts. It is possible that, in the acidic environment, the modified group is released by hydrolysis to return to the hydroxyl group, so that the compound showed the activity. Since the reveromycin A derivatives (Compound 2 and compound 27) do not show any inhibitory effect on the growth of the undifferentiated RAW 264 cells even at a comparative high concentration and show the selective apoptosis inducing effect on the mature osteoclasts, it was shown that the reveromycin A derivatives are potential prodrugs.

Table 3 $ED_{50}$ level (pM) of cell-death inducing activity to mature osteoclasts and undifferentiated RAW264 cells

| | mature osteoclasts | RAW264 cells | | mature osteoclasts | RAW264 cells |
|---|---|---|---|---|---|
| | Number of cells | MTT | | Number of cells | MTT |
| Reveromycin A | 0.18 | 24 | 13 | 2.5 | 7.6 |
| 1 | >45 | 132 | 14 | 3.3 | 25 |
| 2 | 6.6 | 89 | 15. | 5.8 | 25 |
| 3 | >6.0 | >6.0 | 16 | 26 | >142 |
| 4 | >5.6 | >5.6 | 17 | >61 | n. c. |
| 5 | >47 | >157 | 18 | 37 | 44 |
| 1390-II | >5.2 | n. d. | 19 | 36 | >50 |
| 1392-II | >52 | >174 | 20 | >45 | >150 |
| 6 | >45 | >149 | 21 | 7.3 | 21 |

Table continued

| | mature osteoclasts | RAW264 cells | | mature osteoclasts | RAW264 cells |
|---|---|---|---|---|---|
| | Number of cells | MTT | | Number of cells | MTT |
| 7 | >51 | >169 | 22 | 9.3 | 53 |
| 8 | 31 | 100 | 23 | >17 | >17 |
| 9 | >48 | 61 | 24 | >15 | >15 |
| 10 | >54 | >178 | 25 | >17 | >17 |
| 11 | 41 | >178 | 26 | >44 | >148 |
| 12 | 0.6 | 1.0 | 27 | 1.3 | 49 |
| n. d.: no data | | | | | |

<Example 2> Investigation of the inhibitory effect on liberation of $^{45}Ca$ from mouse long bone

The effects of the reveromycin A derivatives on the facilitation of the bone resorption due to the parathyroid hormone (PTH) were measured.

A 19-day pregnant rat was subjected to the hypodermic injection of $[^{45}Ca]CaCl_2$ to label a fetal bone. After 24 hours, the anterior limb long bone was extracted. For an anterior limb long bone, a Dulbecco-modified Eagle medium (DMEM) containing 15% heat-inactivated horse serum and 100 U/ml penicillin was used as a culture medium and then incubated for 72 hours together with a bone resorption factor (PTH $10^{-8}$M, IL-1 0.3 ng/ml, vitamin $D_3$ $10^{-8}$M) and the reveromycin A derivatives. After incubation, $^{45}Ca$ radioactivities in a solution into which calcium of the bone was eluted by 0. 1 N HCl and in the culture solution were measured using a liquid scintillation counter. The ratio of the amount of calcium released into the culture solution to the total amount of calcium (bone resorption activity (%)) was used as an indicator and the effects of the reveromycin A derivatives on the facilitation of the bone resorption due to the PTH was investigated.

Bone resorption activity (%) = 100 x Amount of $^{45}Ca$ released into culture solution / (Amount of $^{45}Ca$ released into culture solution + Amount of $^{45}Ca$ left in bone)

The reveromycin A derivatives (Compound 2 and Compound 27) inhibited the bone resorption induced by the PTH at $10^{-8}$M, $10^{-7}$M, and $10^{-6}$M, in a concentration-dependent manner. In addition, the bone resorption due to vitamin $D_3$ or IL-1 could be also inhibited.

<Example 3> Investigation of inhibitory effects of reveromycin A derivatives on bone resorption due to parathyroid hormone (PTH)

Inhibitory effects of the reveromycin A derivatives on bone resorption due to a parathyroid hormone (PTH) which functioned as the most important bone resorption factor in a living body were investigated.

The bone resorption inhibitory effects of the reveromycin A derivatives were examined by measuring the level of serum calcium released in the blood by the action of bone resorption. SD rats were subjected to an operation of extracting the thyroid glands and the parathyroid glands (TPTX). Next day, a decrease in serum calcium was confirmed and then the continuous infusion of the parathyroid hormone (PTH) was carried out, thereby creating rats each having hypercalcemia by accelerating the bone resorption.

At 12 hours from the initiation of PTH injection, the reveromycin A derivatives (10 mg/50 μl ethanol (EtOH), 100 g bw) or a sodium salt thereof (10 mg/100 μl 0.05N NaOH, 100 g bw) were administered and the level of serum calcium was then measured in time-course for 24 hours.

A remarkable decrease in the level of serum calcium occurred when a SD rat was subjected to the TPTX. Continuous injection of the PTH to the TPTX rat allowed an increase in the level of serum calcium. On the other hand,

any individual received the administration of the reveromycin A derivatives (Compound 2 and Compound 27) (80 mg/kg) showed a significant suppression of an increase in the level of serum calcium due to PTH. It became evident that the reveromycin A derivatives significantly inhibited the bone resorption due to PTH. Furthermore, some of the individuals administered with the reveromycin A derivatives dissolved in EtOH were led to death upon administration. However, administration of the reveromycin A sodium salt caused no death, thus the reveromycin A derivatives showed an effective inhibitory action on bone resorption.

Furthermore, the inhibitory effect of calcitonin, which is clinically applied as a bone resorption inhibiting factor, on bone resorption was also investigated and the difference thereof with the reveromycin A derivatives was studied. As a result, calcitonin showed a shorter inhibitory action on bone resorption while the reveromycin A derivatives (Compound 2 and Compound 27) showed a prolonged inhibitory action on bone resorption.

INDUSTRIAL APPLICABILITY

The present invention provides a novel therapeutic agent for hypercalcemia and bone diseases and a novel compound to be used as an active ingredient of the therapeutic agent.

## Claims

1. A therapeutic agent for hypercalcemia or bone diseases, comprising as an active ingredient a reveromycin A derivative represented by the general formula (I) or a pharmaceutically acceptable salt thereof.

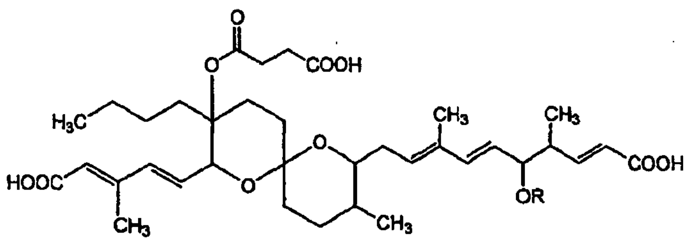

(I)

(R represents a hydroxyl-protecting group releasable under acidic conditions.)

2. The therapeutic agent according to claim 1, wherein R is a tert-butyldimethylsilyl group in the general formula (I).

3. A reveromycin A derivative represented by the general formula (II) or a pharmaceutically acceptable salt thereof.

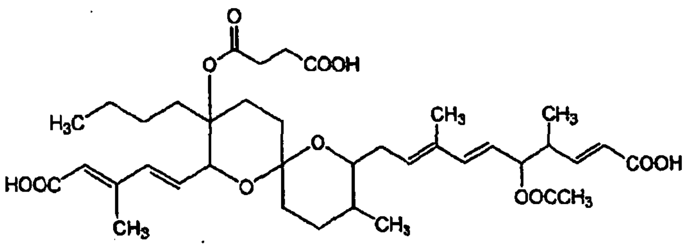

(II)

4. A therapeutic agent for hypercalcemia or bone diseases, comprising as an active ingredient the reveromycin A derivative or the pharmaceutically acceptable salt thereof according to claim 3.

## INTERNATIONAL SEARCH REPORT

International application No.
PCT/JP2004/010125

**A. CLASSIFICATION OF SUBJECT MATTER**
Int.Cl[7] C07D493/10, A61K31/35, A61P3/14, A61P19/08, A61P19/10

According to International Patent Classification (IPC) or to both national classification and IPC

**B. FIELDS SEARCHED**

Minimum documentation searched (classification system followed by classification symbols)
Int.Cl[7] C07D493/10, A61K31/35, A61P3/14, A61P19/08, A61P19/10

Documentation searched other than minimum documentation to the extent that such documents are included in the fields searched

Electronic data base consulted during the international search (name of data base and, where practicable, search terms used)
CAPlus(STN), REGISTRY(STN), BIOSIS(STN), EMBASE(STN), MEDLINE(STN)

**C. DOCUMENTS CONSIDERED TO BE RELEVANT**

| Category* | Citation of document, with indication, where appropriate, of the relevant passages | Relevant to claim No. |
|---|---|---|
| X<br>Y | SHIMIZU, Takeshi et al., "Chemical modification of reveromycin A and its biological activities", Bioorganic & Medicinal Chemistry Letters, 2002, 12(23), pages 3363 to 3366; particularly, Compounds 6, 7, page 3366, lower left column, 6th line to first line from the bottom | 3<br>1,2,4 |
| Y | NAGAI, Kazuo, "Application of molecular probes for studying the process of differentiation and expression of biological functions of the cells participating in bone metabolism", Nippon Nogei Kagaku Kaishi, 2001, 75(2), pages 111 to 119; particularly, page 117, right column, 5th line from the bottom to page 118, left column, line 3 | 1-4 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 17 August, 2004 (17.08.04) | 14 September, 2004 (14.09.04) |

| Name and mailing address of the ISA/ | Authorized officer |
|---|---|
| Japanese Patent Office | |
| Facsimile No. | Telephone No. |

Form PCT/ISA/210 (second sheet) (January 2004)

**INTERNATIONAL SEARCH REPORT**

International application No.
PCT/JP2004/010125

C (Continuation). DOCUMENTS CONSIDERED TO BE RELEVANT

| Category* | Citation of document, with indication, where appropriate, of the relevant passages | Relevant to claim No. |
|---|---|---|
| Y | JP 7-223945 A (Snow Brand Milk Products Co., Ltd.), 22 August, 1995 (22.08.95), (Family: none) | 1-4 |
| Y | WOO, J. et al., "Reveromycin A induces apoptosis in activated osteoclasts, not in inactivated.", Journal of Bone and Mineral Research, 2001, Vol.16, Suppl.1, p.S383 | 1-4 |
| Y | WOO, J. et al., "Reveromycin A induces osteoclast apoptosis and inhibits bone resorption.", Journal of Bone and Mineral Research, 1999, Vol.14, Suppl.1, p.S360 | 1-4 |

Form PCT/ISA/210 (continuation of second sheet) (January 2004)